# EUROPEAN PATENT APPLICATION

(11) **EP 0 608 138 A2**
(43) Date of publication of application: **27.07.1994**
(21) Application number: 94300448.1
(22) Date of filing: 20.01.1994
(51) Int. Cl.: A61B 17/06, B65H 54/56

(54) **Method for winding sutures and wound suture package**

(30) Priority: 21.01.1993 US 6399
(71) Applicant: ETHICON INC., Somerville New Jersey 08876 (US)
(72) Inventor: Hughieson, Alan David, Edinburgh, Midlothian, Scotland (GB); Robertson, James, Grangemouth, Stirlingshire, Scotland (GB)
(74) Representative: Fisher, Adrian John

(57) **Abstract**

A method of winding a suture to provide easy removal of the suture from a package. The suture is wound with a U-bend along its length.

## Description

### Field of the Invention

The present invention relates to an improved method for winding sutures and the resultant wound sutures.

### Background of the Invention

A considerable body of art has been developed on methods forwinding sutures and suture packages. When sutures are to be used, it is important that they be presented for use in a sterile manner. Also, when the suture is removed from the sterile package it should be removed without tangling or knotting. To accomplish this, a number of different winds and/or specifically designed suture packages have been developed to provide a sterile suture package from which the suture can be removed with a minimum of effort and with a minimum possibility of entangling or knotting or getting caught within the package. Examples of such winding techniques or packages are given in U.S. Patents 4,967,902, 5,056,658, 5,099,994, 4,533,401, 4,126,221, and 4,089,409. Examples of packages which provide sutures in a sterile manner and assist in preventing tangling of the suture are given in U.S. Patents 4,884,681, 4,615,435, 4,887,710, and 4,946,043.

While a number of these methods and packages have attained commercial acceptance, they have not overcome all of the problems associated with winding sutures, especially the longer sutures. It is difficult to wind a suture which is 27 inches, 30 inches, 36 inches long or longer in a manner so that when it is removed from the package it does not snag, catch, or kink in the package and disrupt the ease of removal. The object of the present invention provides a method of winding the longer length sutures so they may be readily removed from a suture package. It is a further object of the present invention to provide a package of longer length sutures wherein the suture does not snag, catch or kink upon removal of the suture from the package.

### Summary of the Invention

In accordance with the present invention, there is provided a method of winding a surgical suture for placement in a suture package. In winding the suture, a first portion of the suture is wound in a first direction. The winding is reversed and a second portion of the suture is wound in a second direction opposite to the first direction. Preferably, the suture is wound in an oval configuration. The first half of the suture is wound either clockwise or counter-clockwise and at about the mid-point of the suture, the direction is changed and the remaining portion of the length of the suture is wound in the opposite direction; that is, if the first portion is wound clockwise, then the second portion or remaining portion of the suture is wound counter-clockwise. When such a suture is placed in its final package for sterilization and the like, it will have a sharp U-bend in the suture. Upon removal of such a wound suture from the package, the portion of the suture up to the U-bend is easily and readily removed from the package with the portion of the suture past the U-bend remaining substantially stationary in the package. Once the U-bend is removed from the package the remainder of the suture also is easily removed from the package.

### Brief Description of the Drawings

Figure 1 is a perspective view depicting one method for winding a suture in accordance with the present invention;
Figure 2 is a plan view of a preferred suture holder for packaging a suture wound in accordance with the present invention;
Figure 3 is a plan view of the suture holder of Figure 2 with a wound suture in the holder;
Figure 4 is a plan view of a sterile suture package for a holder having a suture wound in accordance with the present invention disposed therein; and
Figure 5 is a cross-sectional view taken along Line 5-5 of Figure 4.

### Detailed Description of the Drawings

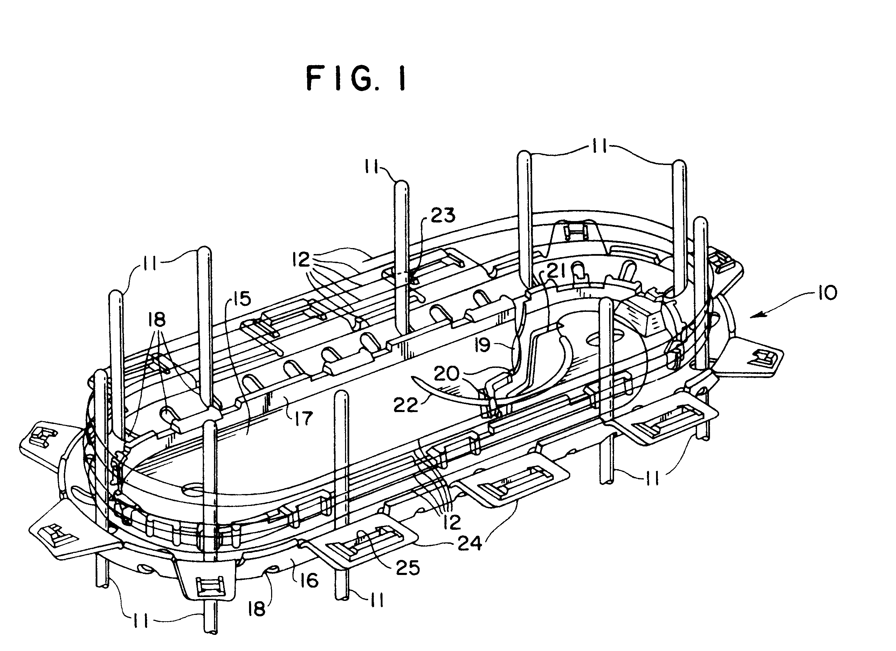

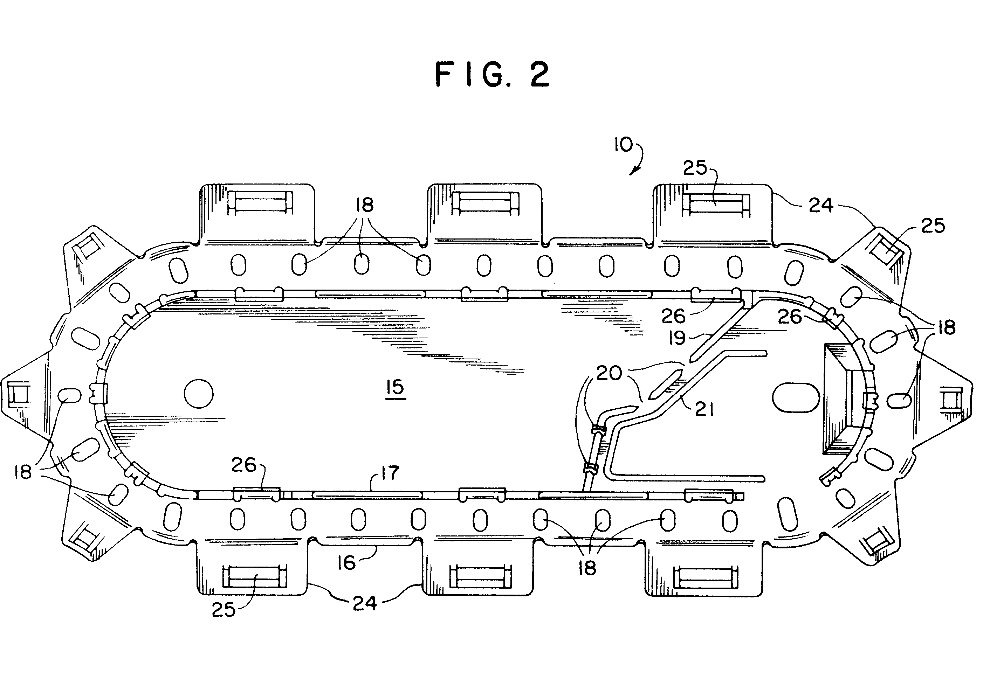

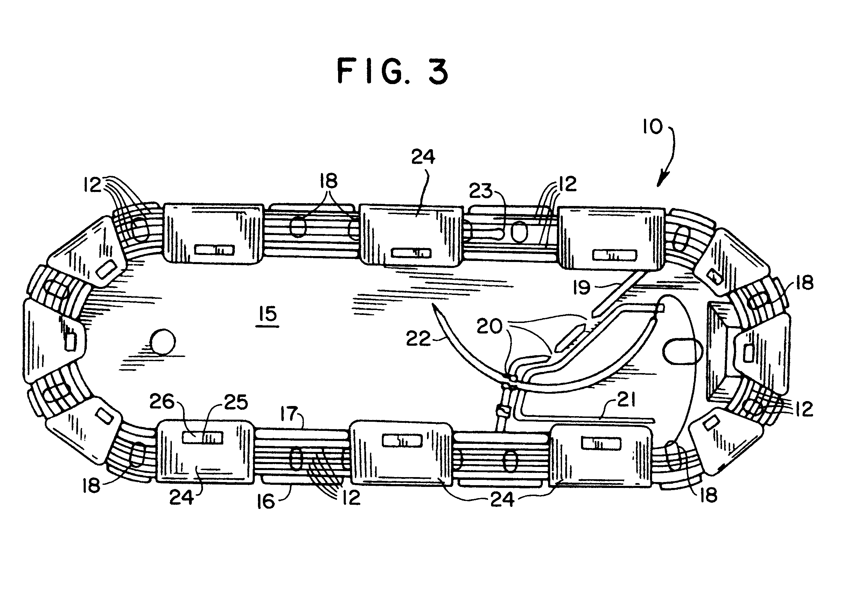

Referring to Figure 1, there is depicted an oval suture holder 10 which has been positioned over a set of pins 11. The pins are retractable. The suture 12 to be placed in the suture holder is wound about the outside of the periphery of the pins and then slipped down into the suture holder and the pins retracted so that the suture holder with the suture disposed therein is ready to be further packaged. Figure 2 is a plan view of the suture holder 10. The suture holder comprises a flat bottom member 15. There is an upstanding vertical wall 16 about the periphery of the holder. Disposed inwardly from that wall is a second vertically upstanding wall 17 substantially parallel to the peripheral wall. These two walls form a channel for holding the suture 12. Disposed in the floor between the two walls is a plurality of openings 18. The openings are used to accept the retractable pins as shown in Figure 1. The retractable pins are used as previously mentioned for winding the suture. Also disposed at one end of the flat bottom and extending substantially between the inwardly disposed wall is a needle holding wall 19. This wall has a plurality of openings 20 in it for accepting the needle. Also, the portion of the suture holder underlying the needle is cut 21 so that it will deflect to allow easy access to the suture by means of a forceps or needle holder. In practicing the method of the present invention, the needle 22 is placed so that it is grasped by the wall on opposite sides of one of the openings. The suture is then wound about the periphery of the pins. Preferably, about half the total length of the suture is wound about the pins in a first direction. In Figure 1 this is the clockwise direction. After about half the length of the suture has been wound, the suture is then reversed and a tight U-turn 23 is formed about one of the pins and the remaining length of the suture is wound on the outside of the pins but now in the opposite direction or in a clockwise direction. After the entire suture has been wound about the pins, the suture is then slipped down to the bottom of the pins and placed into the channel in the suture holder. and the pins retracted. Disposed about the periphery of the suture holder and extending outwardly from the top of the outside wall are a plurality of spaced apart hinged doors 24. As seen in Figure 3, once the suture has been placed in the channel, the hinged doors may be brought to overlie the suture and an opening 25 in the hinge door locks with a tab 26 extending upwardly from the inwardly spaced wall.

As previously mentioned, to remove the suture it is a simple matter to grasp the needle with forceps and draw the suture away from the suture holder. In so doing, the U-bend in the suture and that portion between the U-bend and the needle will be drawn out from the suture holder white the remainder of the suture remains relatively stationary within the suture holder. When the U-bend is finally drawn from the suture holder, then the remaining length of suture beyond the bend will start to move and may be drawn from the suture holder. Unexpectedly, by placing the U-bend somewhere around the U-point of the suture, longer length sutures may be removed from the package without the suture kinking or snagging or getting caught in the suture holder. In prior art packages, when packaging the suture wherein it has been wound all in one direction, if the suture is long, the loops will tend to tighten and bind either on the bend or on the inside wall making it difficult at times to remove the suture from the holder. By placing the U-bend in the suture, this problem of binding or kinking or snagging in the suture holder is eliminated even in the long length sutures. By long length sutures is meant sutures 27 inches, 30 inches, 36 inches or more.

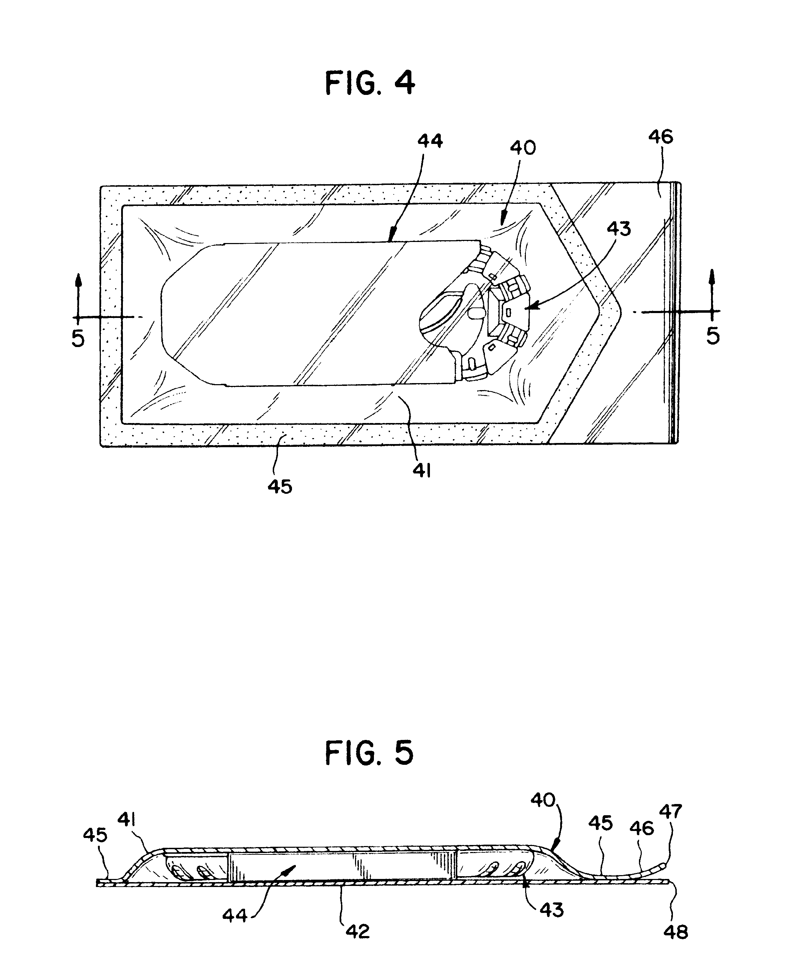

Once the suture and needle have been appropriately placed in a suture holder, the suture holder is further packaged to provide a sterile suture package. Such a package is shown in Figures 4 and 5. The sterile package 40 comprises a top layer 41 and a bottom layer 42 with the suture holder 43 disposed between the top and bottom layer. The majority of the top of the suture holder is covered with a Kraft sulfite board 44 or si mi lar material to help protect the suture. The bottom layer may be made from non-woven, paper, foil or other materials well known in the art and the top layer may be made of similar materials, transparent films and the like. The top and bottom layers 45 are sealed together about the major portion of the periphery and heat sealed together along one side thereof disposed inwardly from the top and bottom layers to provide a grasping area 46 to separate the two layers. Depending on the materials used, the package may be sterilized by radiation, ethylene oxide, or other sterilization techniques. When it is desired to use the steri le package, it is a si mple matter to grasp the non- heat sealed ends 47 and 48 of the top and bottom layer and peel the top layer away from the bottom layer exposing the sterile suture holder for use.

The sutures that may be wound and packaged in accordance with the present invention may be any of the natural or synthetic, absorbable or non-absorbable sutures. Examples of such sutures are silk, catgut, nylon, polypropylene, polydioxanone, polyglyco- lide-lactides, etc. The suture may be braided, multifilament, monofilament, etc. Furthermore, though the invention has been specifically described with regard to a suture and needle, the suture may be double-armed; i.e., a needle at both ends of the suture, or the suture may be without a needle; i.e., unarmed. The suture holder is preferably molded and may be made from any of the standard moldable polymers such as polypropylene and the like.

Though an oval suture holder and wind has been shown, other shapes could also be used such as circular, elliptical, etc.

Having now described the present invention it may be readi ly apparent to those skilled in the art that many variations and modifications may be made without departing from the spirit and scope of the present invention.

## Claims

1. A method of winding a surgical suture for placement of said wound suture in a suture package, said method comprising winding a first portion of said suture in a first direction, reversing said wind and winding a second portion of said suture in a second direction opposite to said first direction.

2. A method according to Claim 1 wherein substantially half the length of the suture is wound in the first direction and the remaining length of suture is wound in the second direction.

3. A method according to Claim 1 wherein the suture is wound in an oval configuration.

4. A method according to Claim 1 wherein the first portion of the suture is wound in a clockwise direction and the second portion of the suture is wound in a counter clockwise direction.

5. A method according to Claim 1 wherein the first portion of the suture is wound in a counter clockwise direction and a second portion of the suture is wound in a clockwise direction.

6. A method according to Claim 1 wherein the suture is wound in an oval configuration with the first portion of the suture being wound in a clockwise direction and a second portion of the suture being wound in a counterclockwise direction.

7. A method according to Claim 6 wherein approximately half of the length of the suture is wound in the clockwise direction and the remaining length of suture is wound in the counter-clockwise direction.

8. A method according to Claim 1 wherein the suture is wound in an oval configuration and the first portion of the suture is wound in a counter- clockwise direction and the second portion of the suture is wound in a clockwise direction.

9. A method according to Claim 7 wherein approximately half of the length of the suture is wound in a counter clockwise direction and the remaining length of suture is wound in the clockwise direction.

10. A suture holder having a wound suture disposed therein, said holder comprising a floor member, a pair of spaced apart vertically upstanding walls, one of said walls disposed about the periphery of said floor member and the other of said walls disposed inwardly from said first wall to form a channel between said walls for holding said suture, a suture configured in a plurality of loops and disposed in said channel at least one of said loops including a U-shaped bend.

11. A suture holder according to Claim 10 wherein the suture holder is oval shaped.

12. A suture holder according to Claim 10 wherein the walls are substantially parallel.

13. A suture holder according to Claim 10 wherein the U-shaped bend in said suture is at approximately the mid-point of said suture.

14. A suture holder according to Claim 10 wherein the holder is an oval holder shape, the other of said walls disposed inwardly from the first wall is discontinuous and is substantially parallel to said first wall and the U-shaped bend in the suture is at approximately the mid-point of said suture.

15. A suture holder according to Claim 10 including means disposed on the floor member of said suture holder for holding a needle attached to said suture.

16. A suture holder according to Claim 10 wherein the floor member includes a third upstanding wall disposed from said floor member on the same side of the floor member as the pair of vertically upstanding walls and said third wall has an opening therein for holding a needle attached to said suture.

17. A suture holder according to Claim 16 wherein the holder has an oval shape, the other of said walls disposed inwardly from said first wall is discontinuous and substantially parallel to said first wall and the U-shaped bend in the suture is approximately at the mid-point of said suture.
